**Europäisches Patentamt**

**European Patent Office**

**Office européen des brevets**

(19)

(11) Veröffentlichungsnummer : **0 158 805**
**B1**

(12) **EUROPÄISCHE PATENTSCHRIFT**

(45) Veröffentlichungstag der Patentschrift :
09.08.89

(51) Int. Cl.⁴ : **B 65 B 55/10**

(21) Anmeldenummer : **85102545.2**

(22) Anmeldetag : **06.03.85**

(54) **Verfahren und Vorrichtung zum Entkeimen von Lebensmittelbehältern.**

(30) Priorität : 14.04.84 DE 3414268
02.11.84 DE 3440014

(43) Veröffentlichungstag der Anmeldung :
23.10.85 Patentblatt 85/43

(45) Bekanntmachung des Hinweises auf die Patenterteilung : 09.08.89 Patentblatt 89/32

(84) Benannte Vertragsstaaten :
CH DE FR GB IT LI SE

(56) Entgegenhaltungen :
DE—A— 3 229 383
FR—A— 2 340 500
GB—A— 2 089 213
US—A— 3 383 831
US—A— 4 296 068

(73) Patentinhaber : **Kolbus GmbH & Co. KG**
**Osnabrücker Strasse 77**
**D-4993 Rahden (DE)**

(72) Erfinder : **Blidschun, Benno**
**Krokusweg 7**
**D-4900 Herford (DE)**
Erfinder : **Lierke, Ernst Günter, Dr. Ing.**
**Westring 13**
**D-6231 Schwalbach (DE)**

EP 0 158 805 B1

## Beschreibung

Die Erfindung betrifft ein Verfahren zum Entkeimen von Lebensmittelbehältern, insbesondere von Bechern aus Kunststoff zur Aufnahme von Milchprodukten, unter Verwendung eines Entkeimungsmittels, das durch Ultraschallvernebelung zu einem Aerosol zerstäubt wird und eine Vorrichtung zur Durchführung des Verfahrens. Ein derartiges Verfahren ist aus der GB-A-2 089 213 bekannt.

Zum Abtöten von Mikroorganismen in Lebensmittelbehältern gibt es verschiedene Entkeimungsverfahren, wie die UV-Bestrahlung, Einblasen eines aufgeheizten Wasserdampf-Luftgemisches und die sogenannte Naß-Aseptik, bei der die Behälterinnenwand mit einem Wasserstoffperoxidgemisch besprüht und durch Heißluft wieder getrocknet wird.

Im Hinblick auf eine möglichst lange Haltbarkeit der Produkte hat sich die Verwendung des Sterilisationsmittels Peroxid als ein sicheres Mikroorganismen abtötendes Mittel erwiesen.

Die keimtötende Wirkung von Peroxid beruht u. a. auf der Bildung von atomarem, während der thermischen Zersetzung besonders aktivem Sauerstoff, der eine starke oxidierende Wirkung im Moment der Entstehung zeigt. Die Zersetzung geschieht außerdem unter katalytischer Mitwirkung organischer Stoffe, z. B. der Mikroorganismen selbst. Nicht zuletzt entsteht der Sterilisiereffekt auch durch das flüssige Wasserstoffperoxid, das in die Zellen der Mikroorganismen eindringt und zerstörend wirkt.

Beim Versprühen des Wasserstoffperoxidgemisches läßt sich lediglich eine relativ geringe Benetzung der Behälterinnenfläche erzielen, da sich mit normalen Sprühdüsen nur Tröpfchen des Entkeimungsmittels von 50 bis 150 μm Durchmesser erzeugen lassen, d. h. beim Versprühen bilden sich auf der Oberfläche der Becher Tropfen, zwischen denen große unbenetzte Flächen verbleiben, die nicht keimfrei sind. Dieses um so mehr, wenn man wenig $H_2O_2$ Lösung (10 bis 30 mg/Becher) versprühen will und die Becher bekanntlich nicht oder schwer benetzbar sind.

Da die Behälter nach dem Sterilisieren nur geringste Mengen an Peroxid aufweisen dürfen, ist eine Entfernung dieses toxischen Stoffes in Form von Abdampfen durch Wärmeeinwirkung notwendig. Der Einsatz des Naß-Aseptik-Verfahrens unter Verwendung eines Wasserstoffperoxidgemisches verlangt folglich stets eine Nachbehandlung zum weitestgehenden Entfernen von Restmengen Peroxid. Insbesondere aber bietet dieses Verfahren keine absolute Sterilisierung der gesamten Becherinnenfläche und letztlich hat das Naß-Aseptik-Verfahren einen verhältnismäßig hohen Sterilisierungsmittel- und auch Energieverbrauch.

Die Aufgabe der Erfindung besteht darin, das bekannte Naß-AseptikVerfahren nebst Vorrichtung derart zu verbessern, daß eine absolut sichere Entkeimung der gesamten Fläche von Lebensmittelbehältern innerhalb kürzester Zeit und bei geringstem Verbrauch von Entkeimungsmitteln und Energie erzielt wird.

Ausgehend von einem Verfahren zum Entkeimen von Lebensmittelbehältern, insbesondere von Bechern aus Kunststoff zur Aufnahme von Milchprodukten, unter Verwendung eines Entkeimungsmittels, das durch Ultraschallvernebelung zu einem Aerosol zerstäubt wird ist das Verfahren nach der Erfindung dadurch gekennzeichnet, daß die durch MHz-Ultraschallvernebelung erzeugte Tröpfchen des Entkeimungsmittels elektrisch geladen und elektrostatisch auf die Flächen eines Behälters niedergeschlagen werden. Weitere Ausgestaltungen des Verfahrens und eine Vorrichtung zur Durchführung des Verfahrens sind in den Unteransprüchen definiert.

Im Gegensatz zu den bisher eingesetzten Entkeimungsverfahren kann gemäß der Erfindung mit Hilfe von ultraschallvernebeltem, elektrostatisch niedergeschlagenem Entkeimungsmittel eine optimale Entkeimung der gesamten Fläche der Behälter erreicht werden. Über das elektrostatische Feld lassen sich die Nebeltröpfchen des Entkeimungsmittels gezielt und in sehr kurzer Zeit auf die zu entkeimende Fläche verbringen. Die theoretisch nicht benetzten Zwickel zwischen den abgeschiedenen Tröpfchen bieten den abzutötenden Mikroorganismen nicht genügend Ausweichmöglichkeit vor dem Entkeimungsmittel. Der beim Zerfall des $H_2O_2$ entstehende atomare Sauerstoff findet also immer in kürzester Entfernung einen abzutötenden Mikroorganismus und kann somit, ehe er zum Sauerstoffmolekül rekombiniert, seine entkeimende Wirkung ausüben. Beim bisher üblichen Aufbringen von Tröpfchen mit einem Durchmesser von 50 bis 150 μm ist die Entfernung des entstehenden atomaren Sauerstoffs bis zum nächsten Mikroorganismus häufig so groß, daß er zu Sauerstoffmolekülen rekombiniert und wirkungslos wird, ehe er sterilisierend wirken kann, die Entkeimung ist dann unvollständig.

Anhand der beigefügten Zeichnung sollen nachfolgend das erfindungsgemäße Verfahren und eine Vorrichtung zur Durchführung desselben beispielsweise erläutert werden. Es zeigen

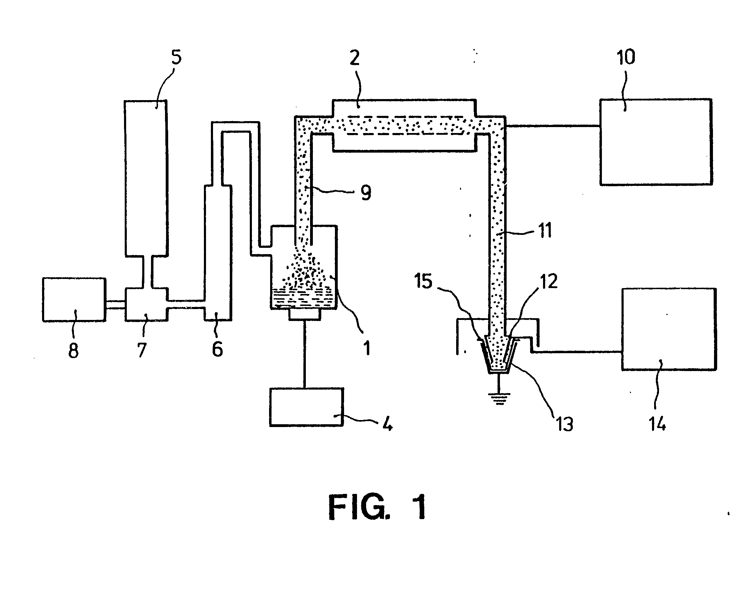

Fig. 1 eine schematische Darstellung der Vorrichtung zum Entkeimen von Lebensmittelbehältern aus Kunststoff unter Verwendung eines Wasserstoffperoxidgemisches,

Fig. 2 eine vergrößerte Darstellung einer Variante der Abscheideeinrichtung,

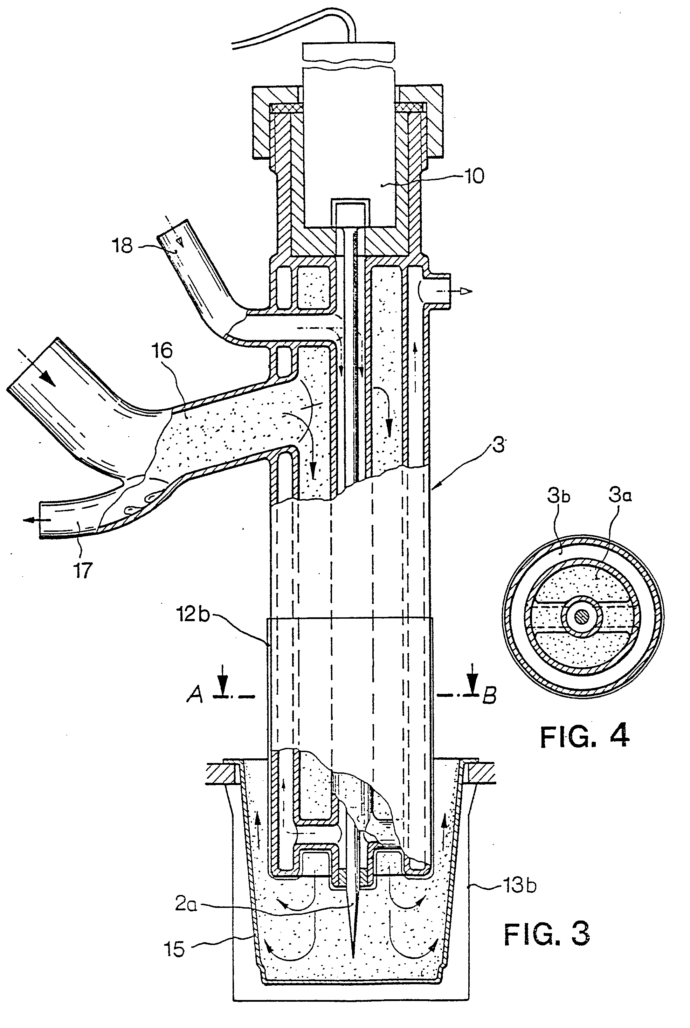

Fig. 3 eine Schnittdarstellung einer weiteren Variante einer Abscheideeinrichtung,

Fig. 4 eine Darstellung gemäß der Schnittlinie A-B in Figur 3.

Ein Aerosolgenerator 1 besteht aus einem mit der zu zerstäubenden Flüssigkeit $H_2O_2$ teilweise gefüllten Gefäß, an dessen Boden ein oder mehrere von einem Generator 4 gespeiste Ultraschallwandler mit einer Arbeitsfrequenz zwischen 1-5 MHz angekoppelt sind.

Die abgestrahlte Schall-Leistung (Schallstrahlungsdruck) dieser Schallwandler führt zu einer Ausbildung von sogenannten Schallsprudeln oder Geysiren, in derem intensiven Schallfeld unter zusätzlicher Verstärkerwirkung schwingender Gasbläschen die Flüssigkeit zu feinen Tröpfchen zerstäubt wird. Bei 1.75 MHz liegt das Maximum der Durchmesser-Häufigkeitsverteilung z. B. bei etwa 3 μm. Der gebildete Nebel wird durch einen Luftstrom aus dem Aerosolgenerator ausgeblasen. Der Luftstrom kann von einem Druckreservoir oder Gebläse 5 geliefert und über einen Impulsgenerator 8 und ein Magnetventil 7 in beliebigem Rhythmus unterbrochen werden. Durch ein Rotameter 6 kann man den Luftstrom messen und auf die optimale Aerosolkonzentration einstellen.

Nach dem Verlassen des Aerosolgenerators durch einen Kamin 9 wird der $H_2O_2$-Nebel im zweiten Rohrabschnitt einer Ladestrecke 2 durch Korona-Aufladung elektrisch aufgeladen. Die Ladungsträger — in der Regel Elektronen — werden durch Spitzenentladung an einer koaxial in die Ladestrecke hineinragenden Elektrode erzeugt. Die erforderliche Feldstärke wird von einem Hochspannungsgenerator 10 (20-50 kV) geliefert.

Bei der Spitzenentladung wird die Umgebungsluft durch die von der negativen Elektrode austretenden Elektronen ionisiert. Positive Gasionen werden von der Spitze abgesaugt, während die Primär- und Sekundärelektronen vom vorbeistreichenden Aerosolstrom eingefangen und mitgenommen werden.

Die geladenen-Aerosoltröpfchen werden am metallisierten Ende 12 einer Rohrstrecke 11 — in der sogenannten Abscheidestrecke — in dem zu sterilisierenden Behälter 15 abgeschieden. Dabei befindet sich der Behälter 15 zwischen zwei konzentrischen Elektroden, von denen die innere, auf negativem Potential liegende Elektrode 12 von einem Generator 14 gespeist wird und die äußere becherförmige Elektrode 13 auf Massepotential liegt. Die negativ geladenen Aerosoltröpfchen treten am Ende der Rohrstrecke aus, passieren den Spalt zwischen Rohrende und Behälterwand und werden dort in dem durch die Becherwand hindurchgreifenden elektrischen Feld abgeschieden. Die vom Generator 14 gelieferte Spannung wird optimal auf die abzuscheidende Aerosolmenge, die elektrische Tröpfchenladung und gewünschte Abscheidezeit abgestimmt.

Beim Abscheiden der Tröpfchen auf der isolierenden Behälterwand baut sich eine Flächenladung auf, die das durch die Wand hindurchgreifende elektrische Feld mehr und mehr abschirmt. Dadurch wird eine gleichmäßige Beschichtung mit Tröpfchen gewährleistet.

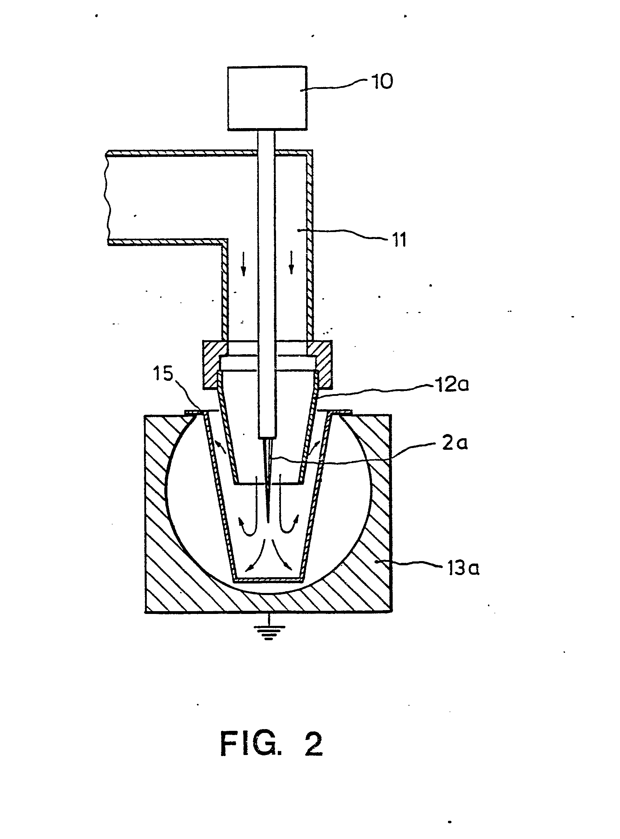

In der Figur 2 wird in einer vergrößerten Darstellung eine weitere Variante der Abscheideeinrichtung gezeigt, in der eine zur Aerosolaufladung dienende Spitzenelektrode 2a Verwendung findet, die direkt in die auf Massepotential liegende äußere, den Behälter 15 umschließende Elektrode 13a hineinragt. Im Hinblick auf eine über die gesamte Behälterwand gleichmäßige Verteilung der Nebeltröpfchen ist der Innenraum der Elektrode 13a in vorteilhafter Weise kugelförmig ausgebildet.

Die Spitzenelektrode 2a wird von einem Generator 10 gespeist und bildet zusammen mit der Elektrode 13a das elektrische Feld in der Abscheideeinrichtung ; die Spitzenelektrode 2a dient folglich gleichzeitig zur Aufladung und Abscheidung der Nebeltröpfchen.

Im oberen Bereich des Behälters 15 befindet sich zwischen der Spitzenelektrode 2a und der Elektrode 13a ferner eine am Ende der Rohrstrecke 11 isoliert aufgehängte Hilfselektrode 12a, die mit der Spitzenelektrode 2a auf gleichem Potential liegt. Die Hilfselektrode wirkt zum einen als Trichter, um die Aerosoltröpfchen in den Wirkbereich der Spitzenelektrode 2a zu lenken, zum anderen aber bildet sie zusammen mit der Elektrode 13a ein zusätzliches elektrisches Feld, das das Entweichen der geladenen Aerosoltröpfchen nach oben verhindert und ein Abscheiden der Tröpfchen im oberen Behälterbereich bewirkt.

In einer weiteren Variante der Abscheideeinrichtung wird diese aus einem Abscheidekopf 3 gebildet, bestehend aus einem schlagfesten Glaskörper mit einer zentral angeordneten Spitzenelektrode 2a, die von einem Hochspannungsgenerator 10 gespeist wird. Die Spitzenelektrode 2a ihrerseits steht mit einer den Glaskörper endseitig umschließenden als Hilfselektrode 12b aufgebrachten Metallfolie in Verbindung. Zwischen der Hilfselektrode 12b und einer Gegenelektrode 13b als Masse, die den Behälter 15 aufnimmt, wird das elektrostatische Feld gebildet, durch das die an der Spitzenelektrode durch Koronaentladung aufgeladenen Tröpfchen des Entkeimungsmittels an der Behälterwand elektrostatisch niedergeschlagen werden. Anstelle der Metallfolie kann auf den endseitigen Teil des Abscheidekopfes 3 ebenso ein Metallfilm als Hilfselektrode 12b aufgebracht werden.

Die in dem Aerosolgenerator 1 mit MHz-Ultraschallwandler kontinuierlich erzeugten Tröpfchen des Entkeimungsmittels werden taktweise über den Abscheidekopf in den Behälter 15 eingebracht, indem ein Luftstrom, mit dem die Tröpfchen aus dem Aerosolbehälter ausgetrieben werden, in vorwählbaren Zeitabständen taktweise zugeführt wird. Über die Taktlänge läßt sich die Abscheidemenge des Entkeimungsmittels steuern.

Der Abscheidekopf 3 hat einen doppelwandigen Glaskörper, in dem ein Strömungskanal 3a für das Entkeimungsmittel von einem mit erwärmter Luft beaufschlagbarem zweiten Strömungskanal 3b umgeben ist. Die auf ca. 50°C erwärmte Luft kann somit über einen Zulauf 18 durch den doppelwandigen Abscheidekopf strömen, wodurch zuverlässig die Bildung von Tröpfchen, die in den Behälter fallen können, verhindert wird. In einer abgewandelten Ausführung kann der Strömungskanal 3a auch von einem Heizelement umgeben sein.

Um in der Zuleitungsstrecke für das Entkeimungsmittel sich bildende Tröpfchen nicht in den Abscheidekopf 3 gelangen zu lassen, ist ein bogenförmig verlaufender, zum Abscheidekopf hin ansteigender Zulauf 16 mit einer an der tiefsten Stelle angeordneten Ableitung 17 vorgesehen.

Abscheidekopf 3 und Gegenelektrode 13b werden über nicht gezeigte Betätigungsmittel taktweise zwischen einer den Behälter 15 zwischen sich einschließenden Arbeitsstellung und einer vom Behälter entfernt liegenden Öffnungsstellung verfahren.

An Stelle des Wasserstoffperoxidgemisches kann das Entkeimungsmittel eine Flüssigkeit wie destilliertes Wasser oder verdünnte Essigsäure sein, die ihre entkeimende Wirkung erst durch chemische Umsetzung infolge der Korona- oder Spitzenentladung erhalten.

Auch bei der elektrostatischen Abscheidung von destilliertem Wasser wird eine Keimabtötung erzielt, wobei durch Luftreaktionen bei der Korona- oder Spitzenentladung Ozon bzw. Stickstoff gebildet wird und diese reaktiven Gase nach Auflösung im Ultraschallnebel und elektrostatischen Abscheidung auf der Becherwandung eine Naß Sterilisation bewirken.

Bei Verwendung von verdünnter Essigsäure oxidiert diese infolge Gasreaktion bei der Korona- oder Spitzenentladung zu Peressigsäure, die schon bei relativ geringen Konzentrationen zur Sterilisation geeignet ist.

Als eine letztmögliche Sicherheitsvorkehrung können die Behälter noch einer Wärmebehandlung unterworfen werden, beispielsweise durch Beaufschlagung der Behälterflächen mit heißer Sterilluft oder durch Mikrowellen.

**Patentansprüche**

1. Verfahren zum Entkeimen von Lebensmittelbehältern, insbesondere von Bechern aus Kunststoff zur Aufnahme von Milchprodukten, unter Verwendung eines Entkeimungsmittels, das durch Ultraschallvernebelung zu einem Aerosol zerstäubt wird, dadurch gekennzeichnet, daß die durch Ultraschallvernebelung erzeugten Tröpfchen des Entkeimungsmittels elektrisch geladen und elektrostatisch auf die Flächen eines Behälters niedergeschlagen werden.

2. Verfahren nach Anspruch 1, dadurch gekennzeichnet, daß durch MHz-Ultraschallvernebelung Tröpfchen mit einem Durchmesser unter 10 μm, vorzugsweise 2-4 μm, erzeugt werden.

3. Verfahren nach Anspruch 1 oder 2, dadurch gekennzeichnet, daß das Entkeimungsmittel dem Behälter über einen Luftstrom zugeführt wird.

4. Verfahren nach Anspruch 1 bis 3, dadurch gekennzeichnet, daß das Entkeimungsmittel dem Behälter über einen nach vorwählbaren Zeitabständen getakteten Luftstrom zugeführt wird.

5. Verfahren nach Anspruch 4, dadurch gekennzeichnet, daß die Abscheidemenge des Entkeimungsmittels über die Taktlänge gesteuert wird.

6. Verfahren nach Anspruch 1 bis 5, dadurch gekennzeichnet, daß die Tröpfchen durch Korona- oder Spitzenentladung geladen werden.

7. Verfahren nach Anspruch 1 bis 6, dadurch gekennzeichnet, daß das Entkeimungsmittel eine Flüssigkeit ist, die durch chemische Umsetzung infolge der Korona- oder Spitzenentladung ihre entkeimende Wirkung verhält.

8. Verfahren nach Anspruch 7, dadurch gekennzeichnet, daß die Flüssigkeit Wasser oder Essigsäure ist.

9. Verfahren nach Anspruch 1 bis 8, dadurch gekennzeichnet, daß das Entkeimungsmittel über einen erwärmten Strömungskanal einer Abscheideeinrichtung dem Behälter zugeführt wird.

10. Verfahren nach Anspruch 1 bis 9, dadurch gekennzeichnet, daß der Behälter im Anschluß an das Entkeimungsverfahren einer Wärmebehandlung unterworfen wird, beispielsweise durch Beaufschlagung der Behälterflächen mit heißer Sterilluft oder durch Mikrowellen.

11. Vorrichtung zur Durchführung des Verfahrens nach einem der Ansprüche 1 bis 10, dadurch gekennzeichnet, daß diese aus einem Aerosolgenerator mit MHz-Ultraschallwandler (1), aus einer elektrischen Aufladeeinrichtung (2, 2a) und aus einer elektrostatischen Abscheideeinrichtung (12, 12a, 12b, 13, 13a, 13b) gebildet ist, der die vom MHz-Ultraschallwandler erzeugten Tröpfchen des Entkeimungsmittels zuführbar sind.

12. Vorrichtung nach Anspruch 11, dadurch gekennzeichnet, daß die elektrische Aufladeeinrichtung (2, 2a) als Korona- oder Spitzenentladungsstrecke ausgestaltet ist.

13. Vorrichtung nach Anspruch 11 oder 12, dadurch gekennzeichnet, daß die Abscheideeinrichtung aus zwei ein elektrisches Feld bildende Elektroden besteht mit einer den Behälter umschließenden außen liegenden Elektrode (13) und mit einer in den Behälter eingreifenden innen liegenden Elektrode (12) in einem definierten Abstand zur Elektrode (13).

14. Vorrichtung nach Anspruch 13, dadurch gekennzeichnet, daß die innen liegende Elektrode (12) der endseitige Teil einer Rohrstrecke (11) ist und koaxial zur außen liegenden Elektrode (13) angeordnet ist.

15. Vorrichtung nach einem der Ansprüche 11 bis 14, dadurch gekennzeichnet, daß die elektrische Aufladeeinrichtung aus einer in eine den Behälter umschließenden außen liegenden Elektrode (13a, 13b) hineinragenden Spitzenelektrode (2a) besteht, und daß die Spitzenelektrode (2a) gleichzeitig mit der außen liegenden Elektrode (13a, 13b) das elektrische Feld in der Abscheideeinrichtung bildet.

16. Vorrichtung nach Anspruch 15, gekennzeichnet durch eine zwischen der Spitzenelektrode (2a) und der außen liegenden Elektrode (13a, 13b) angeordnete Hilfselektrode (12a, 12b), die zusammen mit der außen liegenden Elektrode ein elektrisches Feld bildet.

17. Vorrichtung nach Anspruch 13 bis 16, dadurch gekennzeichnet, daß die außen liegende Elektrode (13, 13a, 13b) einen kugelförmigen Innenraum besitzt.

18. Vorrichtung nach Anspruch 11 bis 17, gekennzeichnet durch einen Abscheidekopf (3) mit einem Strömungskanal (3a), der von einem mit erwärmter Luft beaufschlagbaren zweiten Strömungskanal (3b) umgeben ist.

19. Vorrichtung nach Anspruch 18, dadurch gekennzeichnet, daß der Strömungskanal (3a) des Abscheidekopfes (3) von einem Heizelement umgeben ist..

20. Vorrichtung nach Anspruch 18, gekennzeichnet durch eine mit der Spitzenelektrode (2a) elektrisch leitend verbundene, den endseitigen Teil des Abscheidekopfes (3) umschließenden Hilfselektrode (12b).

21. Vorrichtung nach Anspruch 11 bis 20, dadurch gekennzeichnet, daß der Abscheidekopf (3) und/oder die Gegenelektrode (13, 13a, 13b) taktweise zwischen einer den Behälter zwischen sich einschließenden Arbeitsstellung und einer vom Behälter entfernt liegenden Öffnungsstellung verfahrbar sind.

22. Vorrichtung nach Anspruch 18 bis 21, dadurch gekennzeichnet, daß der Körper des Abscheidekopfes (3) aus einem schlagfesten Glas besteht, dessen endseitiger Teil eine Metallfolie als Hilfselektrode (12b) umgibt.

23. Vorrichtung nach Anspruch 22, dadurch gekennzeichnet, daß die Hilfselektrode (12b) ein auf den endseitigen Teil des Abscheidekopfes (3) aufgebrachter Metallfilm ist.

24. Vorrichtung nach Anspruch 18 bis 23, gekennzeichnet durch einen bogenförmig verlaufenden, zum Abscheidekopf (3) hin ansteigenden Zulauf (16) für das Entkeimungsmittel mit einer an der tiefsten Stelle angeordneten Ableitung (17).

**Claims**

1. Process for sterilizing containers destined to receive foodstuffs, in particular plastic pots for receiving dairy products, said process comprising the use of a sterilizing agent that is ultrasonically atomized so as to form an aerosol, characterized in that the droplets of the sterilizing agent are produced by ultrasonic atomization, are electrically charged, and are electrostatically deposited onto the surfaces of a container.

2. Process according to Claim 1, characterized in that ultrasonic atomization at MHz frequencies is employed to produce droplets with diameters of less than 10 μm, preferably 2-4 μm.

3. Process according to Claim 1 or Claim 2, characterized in that the sterilizing agent is conveyed to the container by means of a stream of air.

4. Process according to any of Claims 1 to 3, characterized in that the sterilizing agent is conveyed to the container by means of a stream of air which is timed to start and stop cyclically at preselectable time intervals.

5. Process according to Claim 4, characterized in that the sterilizing agent is deposited in quantities which are controlled by varying the length of the cyclic time intervals.

6. Process according to any of Claims 1 to 5, characterized in that the droplets are charged by a corona discharge, or by a point discharge.

7. Process according to any of Claims 1 to 6, characterized in that the sterilizing agent is a liquid which retains its effectiveness as a sterilizing agent as a result of undergoing chemical reaction due to the corona or point discharge.

8. Process according to Claim 7, characterized in that the liquid is water or acetic acid.

9. Process according to any of Claims 1 to 8, characterized in that the sterilizing agent is supplied to the container via a heated flow passage belonging to a deposition arrangement.

10. Process according to any of Claims 1 to 9, characterized in that, immediately following the sterilization process, the container is subjected to a heat treatment which may for example be performed by directing hot, sterile air against its surfaces, or by means of microwaves.

11. Appliance for carrying out the process according to any one of Claims 1 to 10, said appliance being characterized in that it is formed by an aerosol generator with a MHz ultrasonic transducer (1), an electric charging device (2, 2a) and an electrostatic deposition arrangement (12, 12a, 12b, 13, 13a, 13b) to which the droplets of the sterilizing agent can be supplied, said droplets being produced by the MHz ultrasonic transducer.

12. Appliance according to Claim 11, characterized in that the electric charging device (2, 2a) is configured as a corona-discharge section, or as a point-discharge section.

13. Appliance according to Claim 11 or Claim 12, characterized in that the deposition arrangement comprises two electrodes which form an electric field, with an outside-located electrode (13) which surrounds the container, and with an inside-located electrode (12) which fits into the container at a defined distance from the electrode (13).

14. Appliance according to Claim 13, characterized in that the inside-located electrode (12) is the end portion of a pipe section (11) and is arranged in a manner such that it is coaxial with the outside-located electrode (13).

15. Appliance according to any one of Claims 11 to 14, characterized in that the electric charging device comprises a point electrode (2a), projecting into an outside-located electrode (13a, 13b) which surrounds the container, and in that the point electrode (2a) forms the electric field in the deposition arrangement, concurrently with the outside-located electrode (13a, 13b).

16. Appliance according to Claim 15, characterized by an auxiliary electrode (12a, 12b) which is positioned between the point electrode (2a) and the outside-located electrode (13a, 13b), and which together with the outside-located electrode forms an electric field.

17. Appliance according to any of Claims 13 to 16, characterized in that the outside-located electrode (13, 13a, 13b) has a spherical internal cavity.

18. Appliance according to any of Claims 11 to 17, characterized by a deposition head (3) with a flow passage (3a) that is surrounded by a second flow passage (3b) to which heated air can be admitted.

19. Appliance according to Claim 18, characterized in that a heating element surrounds the flow passage (3a) belonging to the deposition head (3).

20. Appliance according to Claim 18, characterized by an auxiliary electrode (12b) which surrounds the end portion of the deposition head (3) and is electroconductively connected to the point electrode (2a).

21. Appliance according to any of Claims 11 to 20, characterized in that the deposition head (3) and/or the counter electrode (13, 13a, 13b) can be moved, in a cyclic manner, between an operating position in which they entrap the container between one another, and an opening position distant from the container.

22. Appliance according to any of Claims 18 to 21, characterized in that the body of the deposition head (3) is composed of an impact-resistant glass, its end portion being surrounded by a metal foil which serves as an auxiliary electrode (12b).

23. Appliance according to Claim 22, characterized in that the auxiliary electrode (12b) is a metal film which has been applied to the end portion of the deposition head (3).

24. Appliance according to any of Claims 18 to 23, characterized by an arc-shaped inflow duct (16). for the sterilizing agent, this duct rising towards the deposition head (3) and possessing a drain. facility (17) which is located at the lowest point.

**Revendications**

1. Procédé de stérilisation de récipients pour produits alimentaires, en particulier des godets en matière plastique destinés à recevoir des produits laitiers, avec emploi d'un stérilisant qui est pulvérisé en un aérosol par nébulisation aux ultrasons, caractérisé par le fait que l'on charge électriquement les gouttelettes du stérilisant produites par nébulisation aux ultrasons et qu'on les dépose électrostatiquement sur les surfaces d'un récipient.

2. Procédé selon la revendication 1, caractérisé par le fait que par nébulisation aux ultrasons MHz, on produit des gouttelettes d'un diamètre inférieur à 10 μm, de préférence de 2-4 μm.

3. Procédé selon la revendication 1 ou 2, caractérisé par le fait que l'on amène le stérilisant au récipient au moyen d'un courant d'air.

4. Procédé selon les revendications 1 à 3, caractérisé par le fait que l'on amène le stérilisant au récipient au moyen d'un courant d'air rythmé selon des intervalles de temps prédéterminés.

5. Procédé selon la revendication 4, caractérisé par le fait que l'on commande la quantité de stérilisant déposé par la longueur des rythmes.

6. Procédé selon les revendications 1 à 5, caractérisé par le fait que l'on charge les gouttelettes par décharge par pointe ou par effet de couronne.

7. Procédé selon les revendications 1 à 6, caractérisé par le fait que le stérilisant est un liquide qui acquière sa faculté germicide par transformation chimique à la suite de la décharge par pointe ou par effet de couronne.

8. Procédé selon la revendication 7, caractérisé par le fait que le liquide est de l'eau ou de l'acide acétique.

9. Procédé selon les revendications 1 à 8, caractérisé par le fait que le stérilisant est amené au récipient par un canal d'écoulement chauffé d'un dispositif de précipitation.

10. Procédé selon les revendications 1 à 9, caractérisé par le fait qu'à la suite du procédé de stérilisation, on soumet le récipient à un traitement thermique, par exemple par soufflage d'air stérile chaud sur les surfaces du récipient ou par micro-ondes.

11. Dispositif pour la mise en œuvre du procédé selon l'une des revendications 1 à 10, caractérisé par le fait qu'il est formé par un générateur d'aérosol comportant un transducteur à ultrasons MHz (1), par un dispositif de charge électrique (2, 2a) et par un dispositif de précipitation électrostatique (12, 12a, 12b, 13, 13a, 13b) auquel on délivre les gouttelettes de stérilisant produites par le transducteur à ultrasons MHz.

12. Dispositif selon la revendication 11, caractérisé par le fait que le dispositif de charge électrique (2, 2a) est réalisé sous la forme d'un tronçon de décharge par pointe ou par effet de couronne.

13. Dispositif selon la revendication 11 ou 12, caractérisé par le fait que le dispositif de précipitation se compose de deux électrodes formant un champ électrique, une électrode (13), placée à l'extérieur, entourant le récipient, et une électrode (12) placée à l'intérieur, étant engagée dans le récipient à une distance définie par rapport à l'électrode (13).

14. Dispositif selon la revendication 13, caractérisé par le fait que l'électrode (12), située à l'intérieur, est la partie, côté extrémité, d'un tronçon tubulaire (11) et est disposée coaxialement à l'électrode (13) située à l'extérieur.

15. Dispositif selon l'une des revendications 11 à 14, caractérisé par le fait que le dispositif de charge électrique se compose d'une électrode à pointe (2a) faisant saillie dans une électrode (13a, 13b) située à l'extérieur et entourant le récipient, et que l'électrode à pointe (2a), en liaison avec l'électrode (13a, 13b) située à l'extérieur, forme le champ électrique dans le dispositif de précipitation.

16. Dispositif selon la revendication 15, caractérisé par une électrode auxiliaire (12a, 12b) qui, disposée entre l'électrode à pointe (2a) et l'électrode (13a, 13b) située à l'extérieur, forme un champ électrique en liaison avec l'électrode située à l'extérieur.

17. Dispositif selon les revendications 13 à 16, caractérisé par le fait que l'électrode (13,13a,13b) située à l'extérieur comporte un espace interne en forme de sphère.

18. Dispositif selon les revendications 11 à 17, caractérisé par une tête de précipitation (3) comportant un canal d'écoulement (3a) qui est entouré par un second canal d'écoulement (3b) soumis à l'action d'air chauffé.

19. Dispositif selon la revendication 18, caractérisé par le fait que le canal d'écoulement (3a) de la tête de précipitation (3) est entouré d'un élément chauffant.

20. Dispositif selon la revendication 18, caractérisé par une électrode auxiliaire (12b) raccordée de façon électriquement conductrice à l'électrode à pointe (2a) et entourant la partie, côté extrémité, de la tête de précipitation (3).

21. Dispositif selon les revendications 11 à 20, caractérisé par le fait que la tête de précipitation (3) et/ou la contre-électrode (13, 13a, 13b) peuvent être déplacées de façon rythmée entre une position de travail où elles encerclent le récipient entre elles, et une position d'ouverture située à distance du récipient.

22. Dispositif selon les revendications 18 à 21, caractérisé par le fait que le corps de la tête de précipitation (3) est réalisée en un verre résistant aux chocs dont la partie, côté extrémité, entoure une feuille métallique en tant qu'électrode auxiliaire (12b).

23. Dispositif selon la revendication 22, caractérisé par le fait que l'électrode auxiliaire (12b) est un feuil métallique appliqué sur la partie, côté extrémité, de la tête de précipitation (3).

24. Dispositif selon les revendications 18 à 23, caractérisé par un conduit d'alimentation (16) délivrant le stérilisant, disposé en forme d'arc, allant en montant en direction de la tête de précipitation (3) et comportant un canal d'évacuation (17) placé en son point le plus bas.

FIG. 1

EP 0 158 805 B1

FIG. 2

FIG. 4

FIG. 3